Europäisches Patentamt

European Patent Office

Office européen des brevets

(11) Veröffentlichungsnummer: **0 537 560 A1**

# EUROPÄISCHE PATENTANMELDUNG

(21) Anmeldenummer: **92116810.0**

(22) Anmeldetag: **01.10.92**

(51) Int. Cl.⁵: **C07C 17/00**, C07C 19/08

(30) Priorität: **08.10.91 DE 4133247**

(43) Veröffentlichungstag der Anmeldung:
**21.04.93 Patentblatt 93/16**

(84) Benannte Vertragsstaaten:
**AT BE CH DE DK ES FR GB GR IE IT LI LU NL PT SE**

(71) Anmelder: **Solvay Fluor und Derivate GmbH**
**Hans-Böckler-Allee 20**
**W-3000 Hannover 1(DE)**

(72) Erfinder: **Eicher, Johannes**
**Moorkamp 7**
**W-3008 Garbsen 1(DE)**
Erfinder: **Fazniewscy, Karl-Heinz**
**Im Gesenk 8**
**W-3160 Lehrte(DE)**
Erfinder: **Rieland, Matthias**
**Süsseroder Strasse 4a**
**W-3000 Hannover 71(DE)**
Erfinder: **Rudolph, Werner**
**Oderstrasse 38**
**W-3000 Hannover 71(DE)**

(74) Vertreter: **Lauer, Dieter, Dr.**
**c/o Solvay Deutschland GmbH, Postfach 220**
**W-3000 Hannover (DE)**

(54) **Verfahren zur Herstellung von 1.1.1.2-Tetrafluorethan.**

(57) Beschrieben wird die Herstellung von $CH_2FCF_3$ aus Fluordichlorethylen unter Anwendung einer katalytisch wirkenden Niob-, Tantal- oder Molybdän-Fluorsulfonat-Verbindung oder eines Katalysatorgemisches, welches ein Niob-, Tantal- oder Molybdänhalogenid und ein Sulfonsäurederivat umfaßt.

Rank Xerox (UK) Business Services
(3.10/3.5x/3.0.1)

Die Erfindung bezieht sich auf ein Verfahren zur Herstellung von 1.1.1.2-Tetrafluorethan (R134a), $CH_2FCF_3$.

Es besteht ein zunehmender Bedarf an umweltverträglichen Halogenkohlenwasserstoffen. Als solcher hat sich $CH_2FCF_3$ erwiesen. Diese Verbindung kann beispielsweise als Kältemittel oder Treibmittel eingesetzt werden.

Aufgabe der vorliegenden Erfindung ist es, ein Verfahren zur Herstellung von $CH_2FCF_3$ zur Verfügung zu stellen, das technisch einfach durchzuführen ist und einen hohen Umsatz aufweist.

Diese Aufgabe wird durch das Verfahren der vorliegenden Erfindung unter Verwendung einer katalytisch aktiven Niob, Tantal oder Molybdän enthaltenden Zubereitung gelöst.

Das erfindungsgemäße Verfahren zur Herstellung von $CH_2FCF_3$ ist dadurch gekennzeichnet, daß man $CHFCl=CCl_2$ mit Fluorwasserstoff in flüssiger Phase umsetzt, wobei die Umsetzung durch eine katalytisch aktive Zubereitung, die eine Niob-Fluorsulfonat-Verbindung, eine Tantal-Fluorsulfonat-Verbindung, eine Molybdän-Fluorsulfonat-Verbindung und/oder ein Gemisch aus einem Niob-, Tantal- oder Molybdänpentahalogenid und einem Sulfonsäurederivat, ausgewählt aus der Fluorsulfonsäure und Perfluor(C1-C12)alkansulfonsäure umfassenden Gruppe enthält, katalysiert wird, der Fluorwasserstoff mindestens in der stöchiometrisch notwendigen Menge eingesetzt wird und das Mol-Verhältnis von Ausgangsverbindung zu katalytisch aktiver Verbindung zwischen etwa 10:1 und 1:100 liegt.

Gemäß einer Variante des erfindungsgemäßen Verfahrens verwendet man somit eine Niob-, Tantal- oder Molybdän-Fluorsulfonat-Verbindung. Bevorzugt verwendet man in dieser Variante eine Niob-Fluorsulfonat-Verbindung oder eine Tantal-Fluorsulfonat-Verbindung. Ganz besonders bevorzugt verwendet man eine Verbindung der allgemeinen Formel (I),

$$MX_n(FSO_3)_{5-n} \qquad (I)$$

worin
M = Niob oder Tantal,
X = Halogen und
n = 0 bis 4, vorzugsweise 1 bis 4 bedeutet, als Katalysator. X bedeutet bevorzugt Chlor oder Fluor, und n bedeutet ganz besonders bevorzugt 2 bis 4.

Die Herstellung solcher Verbindungen ist bekannt.

So beschreiben E. Hayek, J. Puschmann und A. Czaloun in Monatshefte Chemie 85 (1954), Seiten 359 bis 363 die Herstellung von $TaCl_3(FSO_3)_2$ aus Tantalpentachlorid und Fluorsulfonsäure und Abdampfen überschüssiger Fluorsulfonsäure. Die Verbindung läßt sich auch aus Tantalpentachlorid und Fluorsulfonsäurederivaten, beispielsweise Fluorsulfonsäureethylester, herstellen. Reaktionsprodukte aus Niobpentachlorid und Fluorsulfonsäure sind viskos oder gallertartig. Nach Ansicht der Autoren H.C. Clark und H.J. Emeleus in J. Chem. Soc. 1958, Seiten 190 bis 195, insbesondere Seite 193, handelt es sich um $NbCl_3(FSO_3)_2$.

Die Autoren H.C. Clark und H.J. Emeleus beschreiben an gleicher Stelle auch die Herstellung von $TaF_3(FSO_3)_2$ und $NbF_3(FSO_3)_2$ aus Tantalpentafluorid bzw. Niobpentafluorid und Schwefeltrioxid bei Raumtemperatur.

Die Autoren W.V. Cicha und F. Aubke beschreiben in J. Fluorine Chem. 47 (1990), Seiten 317 bis 332 die Herstellung von weiteren Tantal- und Niob-Fluorid-FluorsulfonatVerbindungen.

$Ta(FSO_3)_5$ und $Nb(FSO_3)_5$ können durch Oxidation des jeweiligen Metallpulvers mit bis-(Fluorosulfuryl)peroxid in Fluorsulfonsäure hergestellt werden. Bei der Umsetzung von Niobmetallpulver entsteht als Nebenprodukt auch $NbF_2(FSO_3)_3$.

$TaF_4(FSO_3)$ läßt sich beispielsweise durch Ligandenaustauschreaktion zwischen $Ta(FSO_3)_5$ und 4 Moläquivalenten Tantalpentafluorid herstellen.

Gemäß einer besonders bevorzugten Ausführungsform dieser Variante ist die katalytisch aktive Verbindung eine Tantal-Chlorid-Fluorsulfonat-Verbindung oder eine Niob-Chlorid-Fluorsulfonat-Verbindung, die durch die Umsetzung von Tantalpentachlorid oder Niobpentachlorid mit Fluorsulfonsäure im Mol-Verhältnis von 1:1 bis 1:4 erhalten wurde. Anstelle der Fluorsulfonsäure kann man auch Fluorsulfonsäurederivate verwenden, die mit Tantalpentachlorid oder Niobpentachlorid unter Substitution von Chlorid durch Fluorsulfonat reagieren, beispielsweise Fluorsulfonsäureester.

Gemäß einer anderen besonders bevorzugten Ausführungsform dieser Variante verwendet man als katalytisch aktive Verbindung eine Tantal-Fluorid-Fluorsulfonat-Verbindung oder eine Niob-Fluorid-Fluorsulfonat-Verbindung, die durch Umsetzung von Tantalpentafluorid oder Niobpentafluorid mit Schwefeltrioxid erhalten wurde. Alternativ verwendet man entsprechende Verbindungen, die durch Umsetzung von Tantalpentachlorid oder Niobpentachlorid mit Fluorsulfonsäure oder einem Fluorsulfonsäurederivat, welches mit Tantalpentachlorid oder Niobpentachlorid unter Substitution von Chlorid durch Fluorsulfonat reagiert, beispielsweise einem Fluorsulfonsäureester, im Mol-Verhältnis von 1:1 bis 1:4 und anschließendem Chlor-Fluor-Austausch erhalten wurden.

In der vorstehend beschriebenen Variante sind allenfalls noch geringe, unerwünschte Mengen an Pentahalogeniden in der Zubereitung vorhanden. Die Erzeugung der Fluorsulfonat-Verbindung kann gewünschtenfalls in Anwesenheit von $CHF=CCl_2$

erfolgen, das dann als Verdünnungsmittel wirkt.

Gemäß einer anderen Variante des erfindungsgemäßen Verfahrens wird die Umsetzung durch eine katalytisch aktive Zubereitung katalysiert, die ein Gemisch aus Niob-, Tantal- oder Molybdänpentahalogenid und einem Sulfonsäurederivat enthält. Bevorzugte Pentahalogenide sind Niobpentahalogenid und Tantalpentahalogenid, insbesondere Niobpentachlorid, Niobpentafluorid, Tantalpentachlorid oder Tantalpentafluorid. Bevorzugte Sulfonsäurederivate sind Fluorsulfonsäure und perfluorierte (C1-C3)-Sulfonsäuren wie Trifluormethansulfonsäure, insbesondere Fluorsulfonsäure. Bevorzugt liegen in dieser Variante Pentahalogenid und Sulfonsäurederivat im Molverhältnis von 5:1 bis 1:5, insbesondere 1:1 bis 1:5 vor. Die Reihenfolge des Vermischens von $CHF = CCl_2$, HF, Pentahalogenid und Sulfonsäurederivat ist nicht kritisch.

Gemäß einer weiteren Variante der vorliegenden Erfindung kann man die Umsetzung durch eine Zubereitung katalysieren, die eine Niob-, Tantal- oder Molybdän-Fluorsulfonatverbindung, ein Niob-, Tantal- oder Molybdänpentahalogenid und ein Sulfonsäurederivat enthält.

Natürlich kann die Zubereitung auch ein Gemisch von Pentahalogeniden verschiedener der vorstehend genannten Metalle, ein Gemisch verschiedener Sulfonsäurederivate oder ein Gemisch verschiedener Fluorsulfonat-Verbindungen der vorstehend genannten Metalle enthalten.

Das Mol-Verhältnis von Ausgangsverbindung zur Fluorsulfonat-Verbindung bzw. zum Gemisch von Pentahalogenid und Sulfonsäurederivat liegt, wie weiter oben ausgeführt, zwischen 10:1 und 1:100. Sofern ein Katalysatorgemisch vorliegt, wird die Mol-Zahl des Katalysatorgemisches durch Addition der Mol-Zahlen von Metallhalogenid und Sulfonsäurederivat berechnet. Setzt man beispielsweise 1 Mol $CHF = CCl_2$ in Anwesenheit eines Gemisches von 0,5 Mol Niobpentahalogenid und 0,5 Mol Fluorsulfonsäure mit Fluorwasserstoff um, so beträgt das Molverhältnis von Ausgangsverbindung zu Katalysatorgemisch in diesem Falle 1:1.

Bevorzugt liegt das Mol-Verhältnis von Ausgangsverbindung zu Katalysator bzw. Katalysatorgemisch zwischen 10:1 und 1:10, insbesondere zwischen 2:1 und 1:5.

Das Mol-Verhältnis zwischen $CHF = CCl_2$ und Fluorwasserstoff liegt bevorzugt zwischen 1:10 und 1:100.

Die einzusetzende Menge an Fluorwasserstoff kann über die zur Fluorwasserstoffanlagerung und zum Chlor-Fluor-Austausch benötigte Menge hinausgehen. Wenn man nämlich Metallpentachloride oder Metallpentabromide bzw. Metall-Halogenid-Fluorsulfonat-Verbindungen einsetzt, ist anzunehmen, daß mehr oder weniger ein Austausch von Chlor oder Brom gegen Fluor erfolgt. Wenn also beispielsweise Niobpentachlorid oder Tantalpentachlorid oder $NbCl(FSO_3)_4$ bzw. $TaCl(FSO_3)_4$ in der Zubereitung enthalten sind, können diese im Reaktionsgemisch möglicherweise in Form teilfluorierter oder fluorierter Metallverbindungen vorliegen.

Angaben zur einzusetzenden Menge an Fluorwasserstoff sind angesichts der vorstehenden Ausführungen in dem Sinne zu verstehen, daß für jedes in das Substratmolekül eingeführte Fluoratom zweckmäßigerweise Fluorwasserstoff in einer solchen Menge eingesetzt wird, die mindestens der stöchiometrisch notwendigen Menge entspricht, und zusätzlich noch soviel Fluorwasserstoff, wie für einen etwaigen Halogen-Fluor-Austausch des Metallhalogenids benötigt wird. Der Einfachheit halber wird in der vorliegenden Erfindung nicht jedesmal auf diese Definition hingewiesen.

Um abzuschätzen, welche Menge zusätzlich an Fluorwasserstoff für diesen etwaigen Halogen-Fluor-Austausch notwendig wird, kann der Fachmann die Metallhalogenid oder Metallhalogenid-Fluorsulfonatverbindung enthaltende Zubereitung vorab mit Fluorwasserstoff umsetzen. Die Menge an verbrauchtem Fluorwasserstoff und/oder die Menge an gebildetem Halogenwasserstoff ermöglicht es, zu berechnen, welche Menge an Fluorwasserstoff zu der für die Umsetzung des eingesetzten Halogenwasserstoffs hinaus benötigt wird.

Die Temperatur bei der Umsetzung zwischen $CHF = CCl_2$ und HF liegt vorzugsweise zwischen 50 und 200 °C. Der Druck entspricht dem autogenen Druck oder liegt darüber, er beträgt z.B. bis zu 30 bar (abs.). Hierbei werden Druck und Temperatur so gewählt, daß die Umsetzung in der flüssigen Phase abläuft.

Feuchtigkeit wirkt sich im erfindungsgemäßen Verfahrend störend aus. Die Umsetzung wird daher zweckmäßigerweise unter Bedingungen durchgeführt, die verhindern, daß eine schädliche Menge an Wasser in die Reaktionsmischung gelangen kann. Es ist vorteilhaft, im wesentlichen wasserfreien Wasserstoff zu verwenden. Je nach eingesetzter Menge an Fluorwasserstoff kann es empfehlenswert sein, den handelsüblichen Fluorwasserstoff vor seiner Verwendung zu trocknen. Weiterhin ist es empfehlenswert, die verwendete Apparatur in möglichst trockenem Zustand zu halten. Hierzu kann man Leitungen, Reaktionsgefäße, Einrichtungen zur Produktaufarbeitung und -aufbewahrung mit trockenen Gasen spülen, beispielsweise mit trockener Luft oder trockenem Stickstoff-Gas.

Gemäß einer bevorzugten Ausführungsform des erfindungsgemäßen Verfahrens wird das für die Umsetzung benötigte $CHF = CCl_2$ aus $CHFClCCl_2H$ durch HCl-Abspaltung hergestellt. Die HCl-Abspaltung kann thermolytisch mit oder ohne Katalysator oder mit Basen, beispielsweise Alkalihydroxid, gewünschtenfalls in geeigneten Lösungsmitteln wie Alkoholen, durchgeführt werden.

Gemäß einer ganz besonders bevorzugten Ausführungsform wird zunächst aus $CHCl_2CHCl_2$ durch selektive Fluorierung $CHFClCCl_2H$ hergestellt, diese Verbindung wird unter HCl-Abspaltung in $CHF=CCl_2$ überführt, und das erhaltene $CFH=CCl_2$ wird dann nach dem erfindungsgemäßen Verfahren mit Fluorwasserstoff umgesetzt.

Die selektive Fluorierung von $CHCl_2CHCl_2$ wird beispielsweise von I. V. Martynov und Yu. L. Kruglyak in Zh. Obshch. Khim. 35(6), 967 bis 969 (1965); Chemical Abstracts 63 (1965), 11349g beschrieben. Bevorzugtes selektives Fluorierungsmittel ist Antimontrifluorid, dessen katalytische Wirkung durch Zusatz von Antimonpentachlorid noch gefördert werden kann. Die Umsetzung wird günstig bei 130 bis 220 ° C durchgeführt.

Das erfindungsgemäße Verfahren kann im batch-Verfahren oder kontinuierlich durchgeführt werden.

Die Aufarbeitung der Reaktionsprodukte kann durch Leiten durch einen Gaswäscher und anschließende fraktionierte Destillation erfolgen.

Die für die Durchführung verwendete Apparatur sollte gegenüber Fluorwasserstoff, Metallhalogeniden und Fluorsulfonsäurederivaten resistent sein. Zweckmäßig verwendet man Bauteile aus Teflon und speziellen Legierungen wie "Hastelloy$^R$", einer fluorwasserstoffresistenten Nickellegierung.

Das erfindungsgemäße Verfahren zeichnet sich aus durch hohen Umsatz, hohe Selektivität, und es kann vorteilhafterweise in der flüssigen Phase durchgeführt werden. Polymerisation der Ausgangsverbindung wird nicht beobachtet. Arbeitet man nach bevorzugten Ausführungsformen und geht aus von $CHFClCCl_2H$ oder gar von $CHCl_2CHCl_2$, so hat man den weiteren Vorteil, daß das gewünschte Produkt aus besonders billigen und einfach herzustellenden Ausgangschemikalien erzeugt werden kann.

Das folgende Beispiel soll das erfindungsgemäße Verfahren weiter erläutern, ohne es in seinem Umfang einzuschränken.

Beispiel

Beispiel 1:

Herstellung von 1.1.1.2-Tetrafluorethan aus 1.1.2.2.-Tetrachlorethan mit Tantal enthaltender Zubereitung

1.1. Selektive Fluorierung von 1.1.2.2.-Tetrachlorethan

Die selektive Fluorierung wurde in einem 500 ml-Dreihalskolben mit Flügelrührer durchgeführt. Der Dreihalskolben war über eine 20 cm-Vigreux-Kolonne mit einem Liebigkühler, daran angeschlossener Vorlage und einer Kühlfalle verbunden. Die Beheizung erfolgte über ein Ölbad.

168 g 1.1.2.2.-Tetrachlorethan, 107 g $SbF_3$ und 7 g $SbCl_5$ wurden unter Stickstoffspülung in den Dreihalskolben eingebracht. Der Kolbeninhalt wurde dann unter Rühren aufgeheizt. Das Ölbad wurde über eine Zeitdauer von 4,5 Stunden auf eine Temperatur von 205 ° C gebracht. Flüchtige Bestandteile destillierten ab einer Ölbadtemperatur von 180 ° C. Es wurde 108 g Destillat erhalten. Gemäß einem Gaschromatogramm bestand das Destillat zu 31,5 Gew.-% aus $CHFClCHCl_2$. Weiterhin waren 64,1 Gew.-% des Ausgangsmaterial sowie 3,5 Gew.-% $C_2HFCl_4$ enthalten. Das gewünschte Produkt wurde durch eine anschließende Feindestillation isoliert.

1.2. HCl-Abspaltung aus $CHFClCHCl_2$

Die Umsetzung wurde wie von J. Yarwood und W. J. Orville-Thomas, J. Chem. Soc. (1965), Seiten 7481 bis 7499 durchgeführt. Ethanolische Kaliumhydroxid-Lösung wurde vorgelegt und 28 g des in Beispiel 1.1.1 hergestellten 1-Fluor-1.2.2.-Trichlorethan zugetropft. Das gebildete Produkt wurde kontinuierlich abdestilliert. Es wurde dann noch durch fraktionierte Destillation gereinigt. Ausbeute: 17 g $CHF=CCl_2$ (Siedepunkt: 37 ° C).

1.3. Umsetzung von $CHF=CCl_2$ und Fluorwasserstoff zu $CH_2FCF_3$

Verwendete Apparatur:
Verwendet wurde ein Laborautoklav aus V4A-Stahl ( ein mit Chrom, Nickel und Molybdän legierter Stahl). Das Innenvolumen dieses Autoklaven beträgt 0,25 l. Der Autoklav ist mit einem Magnetrührer, einem Tauchrohr, über welches die Ausgangsverbindungen eindosiert werden können, und einem Thermostutzen, über welchen die Innentemperatur gemessen werden kann, ausgerüstet. Der Laborautoklav weist weiterhin einen Gasauslaß auf, der mit einem mit Wasser gefüllten Gaswäscher verbunden ist. Der Gaswäscher seinerseits ist mit einer Tiefkältekondensations-Einrichtung verbunden.

Versuchsdurchführung:
In den Autoklaven wurden 15 g $TaCl_5$ eingebracht und 20 g Fluorwasserstoff (HF) zukondensiert. Die Mischung wurde auf 95 ° C erhitzt und gebildetes HCl und der Überschuß Fluorwasserstoff abgelassen. Dann wurden 15 g $FSO_3H$ und 15,5 g des in Beispiel 1.2. hergestellten $CHF=CCl_2$ sowie 40 g HF zugegeben. Das Reaktionsgemisch wurde dann etwa 2 Stunden lang auf 110 ° C erhitzt. Eine Probe der flüchtigen organischen Verbindungen enthielt 65 % 1.1.1.2.-Tetrafluorethan und 1.1.2.-

Trifluor-1-chlorethan. Die Reaktionsmischung wurde dann noch weitere 3 Stunden auf 150 °C erhitzt. Dann wurden flüchtige Bestandteile abgedampft, durch den Gaswäscher geleitet und die den Gaswäscher verlassenden organischen Bestandteile in der Tiefkältekondensations-Einrichung abgefangen. Gemäß Analyse bestand das kondensierte Produkt aus 92,1 Gew.-% 1.1.1.2.-Tetrafluorethan und 7.9 Gew.-% Trifluorchlorethan (etwa equimolares Gemisch aus R133a und R133b). Durch eine weitere Destillation wurde 1.1.1.2.-Tetrafluorethan in reiner Form isoliert.

**Patentansprüche**

1. Verfahren zur Herstellung von $CH_2FCF_3$, dadurch gekennzeichnet, daß man $CHF=CCl_2$ mit Fluorwasserstoff in flüssiger Phase umsetzt, wobei die Umsetzung durch eine katalytisch aktive Zubereitung, die eine Niob-Fluorsulfonat-Verbindung, eine Tantal-Fluorsulfonat-Verbindung, eine Molybdän-Fluorsulfonat-Verbindung und/oder ein Gemisch aus einem Niob-, Tantal- oder Molybdänpentahalogenid und einem Sulfonsäurederivat, ausgewählt aus der Fluorsulfonsäure und Perfluor(C1-C12)alkansulfonsäure umfassenden Gruppe enthält, katalysiert wird, der Fluorwasserstoff mindestens in der stöchiometrisch notwendigen Menge eingesetzt wird und das Mol-Verhältnis von Ausgangsverbindung zu katalytisch aktiver Verbindung zwischen etwa 10:1 und 1:100 liegt.

2. Verfahren nach Anspruch 1, dadurch gekennzeichnet, daß die Umsetzung durch eine katalytisch aktive Zubereitung katalysiert wird, die als katalytisch aktive Verbindung ein Gemisch von Niob- oder Tantalpentahalogenid und Fluorsulfonsäure enthält.

3. Verfahren nach Anspruch 2, dadurch gekennzeichnet, daß das Mol-Verhältnis von Niob- bzw. Tantalpentahalogenid zu Fluorsulfonsäure zwischen 5:1 und 1:5 liegt.

4. Verfahren nach Anspruch 1, dadurch gekennzeichnet, daß die Umsetzung durch eine katalytisch aktive Zubereitung katalysiert wird, die eine Tantal- oder Niob-Fluorsulfonat-Verbindung der allgemeinen Formel (I) enthält

$$MX_n(FSO_3)_{5-n} \qquad (I)$$

worin
X = Halogen
M = Niob, Tantal
n = 0 bis 4 bedeutet.

5. Verfahren nach einem der vorhergehenden Ansprüche, dadurch gekennzeichnet, daß man bei einer Temperatur zwischen etwa 50 und 200 °C arbeitet.

6. Verfahren nach einem der vorhergehenden Ansprüche, dadurch gekennzeichnet, daß das Mol-Verhältnis von $CHF=CCl_2$ und Fluorwasserstoff zwischen 1 : 10 und 1 : 100 liegt.

7. Verfahren nach einem der vorhergehenden Ansprüche, dadurch gekennzeichnet, daß das Mol-Verhältnis zwischen $CHF = CCl_2$ zur katalytisch aktiven Verbindung bzw. zum Gemisch der katalytisch aktiven Verbindungen zwischen etwa 10:1 und 1:10 liegt.

8. Verfahren nach einem der vorhergehenden Ansprüche, dadurch gekennzeichnet, daß man in einem ersten Schritt $CHF = CCl_2$ durch HCl-Abspaltung aus $CHFClCCl_2H$ herstellt.

9. Verfahren nach Anspruch 8, dadurch gekennzeichnet, daß man zur Herstellung von $CHF = CCl_2$ zunächst $CHCl_2CHCl_2$ selektiv zu $CHFClCCl_2H$ fluoriert und dann HCl abspaltet.

10. Verfahren nach Anspruch 9, dadurch gekennzeichnet, daß man die selektive Fluorierung mit Antimontrifluorid, gegebenenfalls in Anwesenheit katalysefördernder Mengen von Antimonpentachlorid, bewirkt.

Europäisches
Patentamt

**EUROPÄISCHER RECHERCHENBERICHT**

Nummer der Anmeldung

EP     92 11 6810

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| P,X | EP-A-0 451 746 (KALI-CHEMIE) <br> * Seite 2, Zeile 1 - Seite 7, Zeile 28; Ansprüche 1-3,8-12,15-20; Beispiel 7 * <br> --- | 1-3,5-7 | C07C17/00 <br> C07C19/08 |
| A | EP-A-0 348 190 (E. I. DU PONT DE NEMOURS) <br> * Ansprüche * <br> --- | 1-10 | |
| A | EP-A-0 300 724 (E. I. DU PONT DE NEMOURS) <br> * Ansprüche * <br> --- | 1-10 | |
| D,A | JOURNAL OF THE CHEMICAL SOCIETY Dezember 1965, LETCHWORTH GB <br> Seiten 7481 - 7499 <br> J. YARWOOD ET AL. 'Spectroscopic Studies. Part VI. The Infrared and Raman Spectra of 1,1-Dichloro-2-fluoroethylene, 2-Bromo-1,1-dichloroethylene and Bromotrichloroethylene' <br> * Seite 7482, Zeile 10 - Zeile 17 * <br> --- | 8-10 | |
| D,A | CHEMICAL ABSTRACTS, vol. 63, no. 9, 25. Oktober 1965, Columbus, Ohio, US; abstract no. 11349g, <br> I. V. MARTYNOV ET AL. <br> 'Halo-alpha-nitrocarboxylic acids. IX. Derivatives of fluoronitro-acetic acid' <br> Spalte 1 ; <br> & ZH. OBSHCH. KHIM. <br> Bd. 35, Nr. 6, 1965, <br> Seiten 967 - 969 <br> I. V. MARTYNOV ET AL. <br> * Zusammenfassung * <br> ----- | 9-10 | **RECHERCHIERTE SACHGEBIETE (Int. Cl.5)** <br><br> C07C |

Der vorliegende Recherchenbericht wurde für alle Patentansprüche erstellt

| Recherchenort | Abschlußdatum der Recherche | Prüfer |
|---|---|---|
| DEN HAAG | 15 JANUAR 1993 | ZERVAS B. |